(19) Europäisches Patentamt
European Patent Office
Office européen des brevets

(11) EP 4 006 011 B1

(12) **EUROPEAN PATENT SPECIFICATION**

(45) Date of publication and mention of the grant of the patent:
**05.11.2025 Bulletin 2025/45**

(21) Application number: **21210943.3**

(22) Date of filing: **29.11.2021**

(51) International Patent Classification (IPC):
***C07C 273/12*** (2006.01) ***C07D 251/56*** (2006.01)
***C07D 251/60*** (2006.01)

(52) Cooperative Patent Classification (CPC):
**C07D 251/56; C07C 273/12; C07D 251/60**

(54) **INTEGRATED PROCESS FOR THE PRODUCTION OF MELAMINE AND UREA**

INTEGRIERTES VERFAHREN ZUR HERSTELLUNG VON MELAMIN UND HARNSTOFF

PROCÉDÉ INTÉGRÉ POUR LA PRODUCTION DE MÉLAMINE ET D'URÉE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **30.11.2020 IT 202000028937**

(43) Date of publication of application:
**01.06.2022 Bulletin 2022/22**

(73) Proprietor: **Eurotecnica Contractors and Engineers S.p.A.**
**20159 Milan (IT)**

(72) Inventors:
- **DE AMICIS, Alberto**
  **I-20097 San Donato Milanese (MI) (IT)**
- **DI RUOCCO, Giuseppe**
  **I-23900 Lecco (IT)**

(74) Representative: **Bird & Bird Società tra Avvocati S.r.l.**
**Via Porlezza, 12**
**20123 Milano (IT)**

(56) References cited:
**WO-A1-2015/165741 WO-A1-2016/171562**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

## Description

The present invention relates to an integrated process for the production of melamine and urea.

### Field of the invention

In particular, the present invention falls within the technical field of the integration of process technologies for the production of melamine and urea in integrated plants.

As is known in the field of urea and melamine there is a need for providing integrated processes in which the off-gases generated in the synthesis of melamine are fed to the urea plant/process, where they are used as raw material for the production of at least a part of urea.

Processes for the production of melamine can be either of the catalytic type or non-catalytic type and consequently the off-gas stream coming from the melamine synthesis has a pressure ranging from 2 to 120 bars. The off-gas stream can be water-free or it can contain up to 35% by weight of water.

In general, processes for producing urea comprise a urea synthesis section, one or more decomposition sections, a urea concentration section and a process-water treatment section.

The expression "urea synthesis section" refers to a section operating at pressures ranging from 120 to 220 bars, preferably from 130 to 200 bars, more preferably from 135 to 175 bars.

The expression "decomposition sections" refers to one or more sections operating at a pressure lower than the synthesis pressure in which the urea is separated from the carbamate, obtaining, at the outlet from said section, a more concentrated urea solution than at the inlet and a carbamate stream in liquid phase or in gas phase.

### Background of the invention

Urea is produced through two consecutive reactions.

In the first reaction, ammonium carbamate is formed according to the following exothermic reaction:

(a)

$$2NH_3 + CO_2 \rightarrow H_2N\text{-}CO\text{-}ONH_4$$

$\Delta H$ = -117 kJ/mole

In the second reaction, the ammonium carbamate is subsequently dehydrated to give urea according to the following endothermic equilibrium reaction:

(b)

$$H_2N\text{-}CO\text{-}ONH_4 \leftrightarrow H_2N\text{-}CO\text{-}NH_2 + H_2O$$

$\Delta H$ = + 15.5 kJ/mole

Melamine is produced from urea according to the following total reaction:

(c)

$$\underset{\text{UREA}}{6\,(H_2N\text{-}CO\text{-}NH_2)} \rightarrow \underset{\text{MELAMINE}}{C_3H_6N_6} + 6NH_3 + 3CO_2$$

When the urea production and melamine production are integrated, at least part of the urea produced in the synthesis section of the urea plant is sent to the melamine synthesis section of the melamine plant. The off-gas stream, composed of ammonia and carbon dioxide, resulting from the conversion of urea into melamine (reaction c) is recycled as raw material from the melamine plant to the urea plant, more specifically in the urea synthesis section.

The stoichiometric ratio between ammonia and carbon dioxide resulting from the synthesis of melamine is the same stoichiometric ratio required for the synthesis of urea, i.e. a 2:1 ratio. The off-gases deriving from the synthesis of melamine are therefore particularly suitable for being used as raw material in the urea synthesis section.

State of the art

In order to meet the need for efficiently integrated urea and melamine production processes, various solutions have been proposed that use off-gases, i.e. $NH_3$ and $CO_2$ deriving from the synthesis of melamine, as raw material for the production of urea.

A first integration example is described in EP1716111. According to the process described in this document (figures 1 and 2), the off-gases generated by the synthesis of melamine are fed to an off-gas condensation section 17 of the urea plant. Both the off-gas stream coming from the melamine synthesis and the carbamate stream 34 coming from the urea recovery section 16 of the urea plant are fed to this section. The condensation of the melamine off-gases takes place by heat transfer to a cooling fluid 42. The resulting carbamate stream is then fed to the urea synthesis section 15, through section 19 where the stream is compressed at a pressure higher than that of the synthesis pressure.

A second example of an integrated process for the synthesis of melamine and urea is described in EP3083571 and relates to a specific process for $CO_2$ stripping processes with a single low-pressure decomposition section. As shown in Figure 1 of EP3083571, the off-gases from melamine are at the same pressure at which the urea synthesis section operates and are condensed in the high-pressure carbamate condensation section (HPCC). In the embodiment of figure 4, the addition of a condenser for the carbamate (CARBCOND) is provided, which receives the off-gases from the melamine plant and the carbamate from the urea recovery section (LPREC). The condensation takes place by heat transfer to a cooling fluid. In the embodiment of Figure 6, a pre-evaporator (PRE-EVAP) is added which receives the off-gases from the melamine plant and the carbamate from the urea recovery section (LPREC). In this case, the condensation takes place by heat transfer to the urea solution coming from LPREC. Figure 8 shows an embodiment wherein, with respect to the configuration of figure 6, an evaporator (FLASH) is added, whose vapours go to the pre-evaporator (PRE-EVAP) together with the off-gases coming from the melamine plant and the carbamate coming from the urea recovery section (LPREC). The condensation takes place by heat transfer to the urea solution coming from the urea recovery section (LPREC). Finally in the embodiment of figure 10, the high-pressure scrubber (HPSCR) is eliminated from the configuration of figure 8, the vapours from the urea reactor go to the pre-evaporator (PRE-EVAP) together with the off-gases coming from the melamine plant and carbamate from the urea recovery section (LPREC). The condensation takes place by heat transfer to the urea solution from LPREC. An integrated process for the production of urea and melamine is disclosed in WO 2016/171562 A1.

Drawbacks of the state of the art

As indicated above, the carbamate formation reaction is an exothermic reaction which supplies heat to the urea production process.

In urea plants, whatever technology may be adopted, $NH_3$ and $CO_2$ are fed to the urea synthesis section where the formation of carbamate takes place and the heat generated by the carbamate formation reaction (a) starting from $NH_3$ and $CO_2$ is then used in the dehydration reaction to urea (b).

When a part of the raw material to be fed to the urea synthesis section consisting of $NH_3$ and $CO_2$, is replaced by carbamate ($H_2N$-CO-$ONH_4$) obtained by condensation of the off-gases coming from the melamine plant, it is evident that part of the heat deriving from reaction (a) is missing in the urea synthesis section.

Furthermore, as previously specified, in the processes described in the state of the art, the condensation heat of the off-gases coming from the melamine plant into carbamate is completely dissipated to a cooling fluid or is transferred to the urea solution downstream of the urea synthesis section.

The main disadvantage of the integration processes proposed by the state of the art is therefore linked to the fact that the heat recovered from the condensation of off-gases into carbamate is never transferred to the urea synthesis section.

The integration of the urea plant with the melamine plant with the feeding of carbamate in place of $NH_3$ and $CO_2$ to the urea synthesis section, resulting, as already mentioned, in a loss of part of the heat deriving from reaction (a), consequently leads to an increase in steam consumption in the urea synthesis section. The increase in steam consumption is equivalent to the lack of heat generated by reaction (a) which, in the case of the processes proposed in the prior art, is dissipated or transferred downstream of the urea synthesis section.

The objective of the present invention is therefore to identify an integrated process which at the same time provides the production of melamine and the production of urea, with an efficient recycling of the off-gases produced in the melamine synthesis to the urea synthesis section.

A further objective of the present invention is therefore to identify an integrated process that at the same time provides for the production of melamine and the production of urea, overcoming the drawbacks of the state of the art indicated above.

Summary of the invention

These and other objectives are achieved by means of the integrated process according to the present invention.

The present invention therefore relates to an integrated process for the production of urea and melamine as described in claim 1.

More specifically, the present invention relates to an integrated process for the production of urea and melamine comprising the following steps:

i) feeding an off-gas stream (11) coming from the melamine synthesis process to a partial condensation and heat recovery step carried out at the same pressure as the off-gases, obtaining a partially liquid stream of carbamate (13);
ii) total condensation of the partially liquid stream of carbamate (13), obtaining a liquid stream of carbamate (15);
iii) compression of the liquid stream of carbamate (15) obtained from step ii) at a pressure equal to or higher than the urea synthesis pressure (130-200 bars), obtaining a liquid stream of compressed carbamate (16);
iv) the liquid stream of compressed carbamate (16) coming from step iii) is put in contact with the off-gas stream (11) coming from the melamine synthesis process with consequent heat exchange wherein the heat produced by the partial condensation of the off-gas stream (11) being fed is transferred to the liquid stream of compressed carbamate (16) and;
v) feeding said liquid stream of compressed and heated carbamate (20) obtained in step iv) as reagent of the urea synthesis process.

The integrated process for the production of urea and melamine according to the present invention thus allows the above-mentioned problems to be solved and above all the problem of the heat balance of the urea synthesis section.

The off-gases from the melamine plant are composed of $NH_3$ and $CO_2$. Depending on the technology applied in the melamine plant, they contain from 0 to 35% by weight of water and the pressure varies from 2 to 120 bars.

The condensation of the off-gases is necessary as the pressure at which the off-gases are available is lower than the urea synthesis pressure.

In one embodiment, the process according to the present invention also comprises a step i') following step i), wherein the partially liquid stream of carbamate (13) coming from step i) heats a stream of ammonia (17), subsequently fed as reagent (18) to the urea synthesis process.

Step i') can coincide with step ii) for the total condensation of the partially liquid stream of carbamate (13), and specifically, in this embodiment, the total condensation of the partially liquid stream of carbamate (13) is effected by heat transfer to the ammonia stream (17), obtaining a liquid stream of carbamate (15) and a heated stream of ammonia (18), subsequently fed as reagent (18) to the urea synthesis process.

Alternatively, step ii) for the total condensation of the partially liquid stream of carbamate (13), can be carried out by transferring heat to a cooling fluid (21), thus obtaining the liquid stream of carbamate (15).

Finally, step ii) for the total condensation of the partially liquid stream of carbamate (13), can be carried out by means of

- a first partial heat transfer to a stream of ammonia (17), obtaining a heated ammonia stream (18), subsequently fed as reagent (18) to the urea synthesis process, and a still partially liquid stream of carbamate ( 14);
  and

- a second heat transfer from the still partially liquid stream of carbamate (14) to a cooling fluid (21), thus obtaining the liquid stream of carbamate (15).

The water necessary for the condensation of $NH_3$ and $CO_2$, at the condensation pressure, may already be present in the off-gases coming from the melamine process as described above.

The water necessary for the condensation of $NH_3$ and $CO_2$ depends on the condensation pressure and the ammonia/carbon dioxide ratio and more specifically, the water required for the condensation decreases with an increase in the condensation pressure and with an increase in the $NH_3/CO_2$ ratio.

For a $NH_3/CO_2$ = 2 ratio, as the pressure increases, the water necessary for the condensation is:

P = 20 bar g Water 20.0% by weight
P = 30 bar g Water 16.4% by weight
P = 40 bar g Water 14.2% by weight

For a $NH_3/CO_2$ = 3 ratio, as the pressure increases, the water necessary for the condensation is

P = 20 bar g Water 19.3% by weight
P = 30 bar g Water 14.8% by weight
P = 40 bar g Water 10.4% by weight

In the case therefore of anhydrous off-gases or with a water content lower than that necessary for obtaining the total condensation of the off-gases, the process according to the present invention also comprises feeding an aqueous stream (12), coming from the urea plant, to the off-gas stream. The aqueous stream (12) is preferably an aqueous solution of carbamate coming from one of the decomposition sections of the urea process, or an aqueous solution of carbamate coming from the process-water treatment section of the urea process.

As already indicated, the process according to the present invention can also comprise a step i') wherein an ammonia stream is preheated with a partially liquid stream of carbamate coming from step i) and the pre-heated ammonia stream is fed to the synthesis of urea, whereas the partially liquid stream of carbamate, once the ammonia has been pre-heated, is fed to the condensation step ii) of the process according to the present invention.

A further object of the present invention relates to an apparatus for the integration of a plant for the production of urea and a plant for the production of melamine which comprises a heat recovery block (1'), connected by means of a line (13') to a condensation block (2'), in turn connected by means of a line (15') to a compression block (3'), in turn connected by means of a supply duct (16') to the heat recovery block (1'), said heat recovery block (1') being fed by a supply duct of off-gases (11') coming from the melamine production plant and comprising an outlet duct (20') suitable for feeding a liquid stream of compressed and heated carbamate to the synthesis section of the urea plant.

The heat recovery block (1') is connected by means of a supply duct (13') to the condensation block (2'), in turn connected to the compression block (3'), in turn connected by means of a supply duct (16') to the heat recovery block (1').

Said apparatus comprises a condensation block (2'), positioned downstream and connected by means of a line (13') to the heat recovery block (1') and positioned upstream and connected by means of a line (15') to the compression block (3'), with respect to the flow direction of the off-gases and the carbamate stream.

In the present description, the terms "upstream" or "downstream" always refer to the flow direction of the off-gases and carbamate streams.

The process object of the present invention is described hereunder with reference to figure 1:
the off-gases (11) coming from the melamine plant are fed to a heat recovery unit (1) which operates at the pressure of the off-gases coming from the melamine plant. The partially liquid stream of carbamate (13) obtained by the partial condensation of the off-gases, leaving the heat recovery block (1'), is completely condensed in a condensation block (2) and the liquid carbamate stream (15) is then compressed in a compression block (3) at a pressure equal to or greater than the urea synthesis pressure and fed back as a liquid stream of compressed carbamate (16) to the same heat recovery block (1'), where heat exchange takes place and the heat produced by the partial condensation of the off-gas stream (11) in the feed is transferred to the liquid stream of compressed carbamate (16). The compressed and heated liquid stream of carbamate (20), leaving the heat recovery block (1'), is then fed to the urea synthesis section of the urea plant.

Complete condensation in the condensation block (2') can take place by heat transfer to an ammonia stream (17) or to a cooling fluid (21) or to both an ammonia stream (17) and a cooling fluid (21) .

Figures 1 and 2 show the embodiment wherein the stream entering the condenser block (2') can be an ammonia stream (17) or a stream of cooling fluid (21).

Steps i) and iv) of the process according to the present invention take place in the heat recovery block (1'): the reaction (a) for the formation of the carbamate described above then takes place, together with the heat transfer for the formation of the carbamate obtained by the partial condensation of the off-gases to the liquid and compressed carbamate stream, thus heated and fed to the urea synthesis.

With the process according to the present invention, the heat not supplied to the urea synthesis with the feeding of fresh $NH_3$ and $CO_2$ is then partially supplied by preheating the carbamate stream fed in the liquid phase to the urea synthesis section and possibly also preheating a stream of ammonia fed to the urea synthesis section.

As already specified, the water necessary for the condensation of $NH_3$ and $CO_2$, at the condensation pressure, can already be present in the off-gases coming from the melamine process or alternatively, in the case of anhydrous off-gases or with a lower water content with respect to that necessary for obtaining the total condensation of the off-gases, the process according to the present invention can comprise a step for feeding an aqueous stream coming from the urea plant, also comprising feeding an aqueous stream (12) coming from the urea plant, upstream of the condensation step ii).

This embodiment is shown in figure 2, where an auxiliary aqueous stream (12) fed upstream of the condenser (2') is represented, which provides the water necessary for the complete condensation of the off-gases.

The auxiliary aqueous stream (12), coming from the urea plant to which the off-gases deriving from the synthesis of melamine are recycled, can be an aqueous solution of carbamate coming from one of the decomposition sections of the urea process, or an aqueous solution of carbamate coming from the process-water treatment section again of the urea process.

A possible further variant of the process scheme according to the present invention represented in figures 1 and

2, is illustrated in figure 3.

In the diagram of figure 3, an ammonia stream (17) is preheated in a preheater (4’) with the partially liquid stream of carbamate (13) coming from the heat recovery (1’) and the pre-heated ammonia stream (18) is then fed to the urea synthesis section. The still partially liquid stream of carbamate (14) leaving the preheater (4’) is then fed to the condenser (2’), where it transfers heat to a cooling fluid (21), thus forming the liquid stream of carbamate (15), similarly to what is also shown in figure 1.

The apparatus according to the invention can therefore also comprise a preheater (4’), positioned downstream of the heat recovery block (1’), with respect to the flow direction of the off-gases and the carbamate stream, and connected to said heat recovery block (1’) by means of a duct (13’) for feeding a partially condensed carbamate stream, said preheater (4’) being fed by means of a duct (17’) suitable for supplying a stream of ammonia and comprising an outlet duct (18’) suitable for feeding the ammonia stream preheated in said preheater (4’) to the urea synthesis section and an outlet duct (14’) suitable for feeding a partially liquid stream of carbamate to the condensation block (2’).

The apparatus according to the invention can also comprise a supply duct (12’) suitable for feeding an aqueous stream upstream (figure 4) or downstream (figure 2) of the heat recovery block (1’), with respect to the flow direction of the off-gases and the carbamate stream.

Furthermore, in the apparatus according to the invention, the supply duct (12’) suitable for feeding the aqueous stream can be connected upstream or downstream (figure 4) of the preheater (4’) and upstream of the condenser (2’).

Through the process scheme shown in both figure 1 and figure 2 (where the auxiliary stream (12) described above is also provided), the heat recovered from the formation of carbamate by condensation of the off-gases is divided into two streams:

- a pre-heated ammonia stream (18) and a compressed and heated liquid carbamate stream (20), both fed to the urea synthesis section; or
- a cooling fluid (22) and a liquid stream of compressed and heated carbamate (20), said carbamate stream (20) being fed to the urea synthesis section.

The first solution is preferable as it involves a complete recovery of the condensation heat of the off-gases which is thus transferred to the urea synthesis section.

Through the process scheme shown in both figure 3 and figure 4 (where the auxiliary stream (12) described above is also provided), the heat recovered from the formation of carbamate by condensation of the off-gases is divided into three streams, a stream of pre-heated ammonia (18) and a liquid stream of compressed and heated carbamate (20), both fed to the urea synthesis section and a cooling fluid (22).

In the embodiment comprising the preheating of the ammonia stream (17), the process according to the present invention therefore has the further advantage of preheating the stream (17) and eliminating or minimizing the heat transferred to the cooling fluid (21) in the condensation block (2’). The process is also characterized by a greater versatility, suppling heat to the urea synthesis both through the liquid stream of compressed and heated carbamate (20) and through the pre-heated ammonia stream (18).

As already specified, in the case of anhydrous off-gases or with a water content lower than that required for obtaining condensation, the auxiliary stream (12) provides the water necessary for the total condensation. This stream (12) can be fed to the off-gas stream fed to step i) of the process according to the present invention before or after the pre-heating step i’) of the ammonia stream. That is, this feeding takes place upstream or downstream of step i’) for heating the ammonia stream (17) with said off-gas stream (11, 13), and upstream of the condensation step ii).

All the embodiments of the process according to the present invention previously described, allow the significant advantage of recycling the formation heat of the carbamate obtained by condensation of the off-gases coming from the ’melamine synthesis plant, to the urea synthesis section, in a particularly effective way.

The process according to the present invention consequently allows the steam consumption to be reduced in the urea synthesis section when this section is at least partly fed with the off-gases coming from the melamine process.

Not increasing the steam consumption in the urea synthesis section leads to the further advantage, in the case of urea plants existing before the implementation of the melamine plant, of not having to modify the existing equipment. An increase in steam consumption would in fact require an increase in the exchange surfaces of the equipment in the urea synthesis section with a consequent increase in the investment cost due to integration with the melamine plant. By reducing the steam consumption, the process according to the present invention, allows the changes in the urea synthesis section to be minimized or cancelled.

Some embodiment examples of the process according to the present invention are provided hereunder by way of non-limiting example of the present invention,

Example 1

An example of the process according to the present invention was applied for the integration of a melamine plant having a capacity of 180 tons/day with a urea plant having a capacity of 2,000 tons/day.

The off-gases coming from the melamine plant have the following composition by weight: $NH_3$ 43%, $CO_2$ 37%, $H_2O$ 20% and the temperature of the off-gas stream is about 160°C and the pressure about 20 bar g .

The off-gas stream was fed to a first exchanger or heat recovery block (1'), where it was partially condensed and the condensation heat, corresponding to about 1,535,000 kcal/h, was transferred to a stream of liquid and compressed carbamate, coming from the compression group (3'). The partially condensed off-gas stream (or partially liquid carbamate stream) was then fed to a preheater (4'), where the partially condensed off-gas stream was further condensed, transferring heat to a high-pressure ammonia stream, then fed to the synthesis section of the urea plant. The high-pressure ammonia stream (160 bars) at 42,000 kg/h was preheated from an initial temperature of 20°C to a final temperature of 144°C due to the further condensation of the off-gases.

The heat transferred to the ammonia by the above-mentioned condensation of the off-gases proved to correspond to approximately 6,826,000 Kcal/h. The partially liquid carbamate stream leaving the preheater (4') was then condensed completely in a heat exchanger or condenser (2') with the use of a cooling fluid, obtaining a liquid carbamate stream.

As indicated above, the condensed off-gas stream or liquid carbamate stream, after its pressure had been increased to 160 barg in a compression block (3), was fed to the heat exchanger or heat recovery block (1') where it received the heat released by the partial condensation of the off-gases corresponding to about 1,535,000 kcal/h.

In this example the heat recovered from the off-gas condensation and transferred to the urea synthesis is the sum of the heat transferred to the preheated ammonia stream and the preheated compressed liquid carbamate stream, both of which are sent to the urea synthesis.

The heat fed to the urea synthesis section with said carbamate stream and said ammonia stream was equivalent to 1,535,000 + 6,826,000 = 8,361,000 kcal/ h.

The steam used in the urea synthesis is typically saturated steam at 22 bar g, with a latent heat of about 450 kcal/kg.

By converting the heat transferred with the ammonia and carbamate streams into saturated steam, the following is obtained:

$$8.361.000\frac{kcal}{h} \Big/ 450\ \frac{kcal}{kg} = 19.180\ \text{kg/h}$$

This example thus allowed a saving of about 19,180 kg/h of steam in the urea synthesis section, by adopting the procedure/apparatus scheme object of this patent application.

Example 2

A second example of the process according to the present invention was applied for the integration of a melamine plant having a capacity of 180 tons per day with a urea plant having a capacity of 3,000 tons per day.

The off-gases coming from the melamine plant have the following composition by weight: $NH_3$ 43%, $CO_2$ 37%, $H_2O$ 20% and the temperature of the off-gas stream is about 160°C and the pressure about 20 bar g.

The off-gas stream was fed to a first exchanger or heat recovery block (1'), where it was partially condensed and the condensation heat, corresponding to about 1,535,000 kcal/h, was transferred to a stream of liquid and compressed carbamate, coming from the compression group (3'). The partially condensed off-gas stream (or partially liquid carbamate stream) was then fed to a preheater (4'), where the partially condensed off-gas stream was further condensed, transferring heat to a high-pressure ammonia stream, then fed to the synthesis section of the urea plant. The high-pressure ammonia stream (160 bars) at 71,000 kg/h was thus preheated from an initial temperature of 20°C to a final temperature of 132°C due to the condensation of the off-gases.

The heat transferred to the ammonia by the above-mentioned condensation of the off-gases proved to correspond to about 9,704,000 Kcal/h. The carbamate stream leaving the preheater (4') is completely condensed and the use of a cooling fluid for completing the condensation is therefore not necessary.

As indicated above, the stream of condensed off-gases or liquid carbamate stream, after its pressure had been raised to 160 barg in a compression block (3'), was fed to the heat exchanger or heat recovery block (1') where it received the heat released by the partial condensation of the off-gases corresponding to about 1,535,000 kcal/h.

In this example the heat recovered from the condensation of the off-gases and transferred to the urea synthesis is the sum of the heat transferred to the preheated ammonia stream and the preheated compressed liquid carbamate stream,

both sent to the urea synthesis.

The heat fed to the urea synthesis section with said carbamate stream and said ammonia stream was equivalent to 1,535,000 + 9,704,000 = 11,239,000 kcal/h.

In this example, all the heat contained in the off-gas stream 11 was transferred to the urea synthesis with the two streams of carbamate and ammonia.

The steam used in the urea synthesis is typically saturated steam at 22 bar g, with a latent heat of about 450 kcal/kg.

By converting the heat transferred with the ammonia and carbamate streams into saturated steam, the following is obtained:

$$11.239.000\frac{kcal}{h}\Big/\ 450\ \frac{kcal}{kg} = 24.975\ \text{kg/h}$$

This example allowed a saving of about 24,975 kg/h of steam in the urea synthesis section, by adopting the procedure/apparatus scheme object of this patent application. In the said examples 1 and 2, 1 ton/day = 0.011 kg/s, 1 kcal/h = 1.16 J/s, 1 bar g is about 2 bar absolute and 1 kcal/h = 0.001163 kilowatt.

## Claims

**1.** An integrated process for the production of urea and melamine comprising the following steps:

i) feeding an off-gas stream (11) coming from the melamine synthesis process to a partial condensation and heat recovery step carried out at the same pressure as the off-gases, obtaining a partially liquid stream of carbamate (13);
ii) total condensation of the partially liquid stream of carbamate (13), obtaining a liquid stream of carbamate (15);
iii) compression of the liquid stream of carbamate (15) obtained from step ii) at a pressure equal to or higher than the urea synthesis pressure (130-200 bars) obtaining a liquid stream of compressed carbamate (16);
iv) the liquid stream of compressed carbamate (16) coming from step iii) is put in contact with the off-gas stream (11) coming from the melamine synthesis process with consequent heat exchange wherein the heat produced by the partial condensation of the off-gas stream (11) being fed is transferred to the liquid stream of compressed carbamate (16) and;
v) feeding said liquid stream of compressed and heated carbamate (20) obtained in step iv) as reagent of the urea synthesis process.

**2.** The process according to claim 1, wherein step ii) for the total condensation of the partially liquid stream of carbamate (13), is carried out by heat transfer to an ammonia stream (17), obtaining a heated stream of ammonia (18) and a liquid stream of carbamate (15) or to a cooling fluid (21), obtaining a liquid stream of carbamate (15).

**3.** The process according to claim 1, wherein step ii) for the total condensation of the partially liquid stream of carbamate (13), is carried out by means of

- a step i') which comprises a first partial heat transfer to an ammonia stream (17), obtaining a heated ammonia stream (18), subsequently fed as a reagent (18) to the urea synthesis process, and a still partially liquid stream of carbamate (14); and
- a second heat transfer from the still partially liquid stream of carbamate (14) to a cooling fluid (21), obtaining the liquid stream of carbamate (15).

**4.** The process according to one or more of the previous claims, wherein the off-gases (11) contain from 0 to 35% by weight of water and have a pressure ranging from 2 to 120 bars.

**5.** The process according to one or more of the previous claims, also comprising the feeding of an aqueous stream (12) coming from the urea plant, upstream of the condensation step ii).

**6.** The process according to claim 5, wherein the aqueous stream (12) is an aqueous solution of carbamate coming from one of the decomposition sections of the urea process, or an aqueous solution of carbamate coming from the treatment section of the process water, again of the urea process.

7. The process according to one or more of the previous claims from 2 to 6, wherein the feeding of the aqueous stream (12) coming from the urea plant takes place upstream or downstream of step i') for heating the ammonia stream (17) with the off-gas stream (11, 13), and upstream of the condensation step ii).

8. An apparatus for the integration of a plant for the production of urea and a plant for the production of melamine which comprises a heat recovery block (1'), connected by a line (13') to a condensation block (2'), in turn connected by means of a line (15') to a compression block (3'), in turn connected by means of a supply duct (16') to the heat recovery block (1'), said heat recovery block (1') being fed by a supply duct (11') of off-gases coming from the melamine production plant and comprising an outlet duct (20') suitable for feeding a liquid stream of compressed and heated carbamate to the synthesis section of the urea plant.

9. The apparatus according to claim 8, also comprising a preheater (4'), arranged downstream of the heat recovery block (1'), with respect to the flow direction of the off-gases and the carbamate stream, and connected to said heat recovery block (1') by means of a duct (13') for feeding a partially condensed carbamate stream, said preheater (4') being fed by means of a duct (17') suitable for feeding a stream of ammonia and comprising an outlet duct (18') suitable for feeding the stream of ammonia preheated in said preheater (4') to the urea synthesis section and an outlet duct (14') suitable for feeding a partially liquid stream of carbamate to the condensation block (2').

10. The apparatus according to claim 8 or claim 9, wherein the apparatus comprises a supply duct (12') suitable for feeding an aqueous stream upstream or downstream of the heat recovery block (1'), with respect to the flow direction of the off-gases and carbamate stream.

11. The apparatus according to claims 8 and 9, wherein the supply duct (12') suitable for feeding the aqueous stream is connected upstream or downstream of the preheater (4') and upstream of the condenser (2').

**Patentansprüche**

1. Integriertes Verfahren zur Herstellung von Harnstoff und Melamin, umfassend die folgenden Schritte:

   i) Zuführen eines aus dem Melaminsyntheseverfahren stammenden Abgasstroms (11) zu einem Teilkondensations- und Wärmerückgewinnungsschritt, der bei demselben Druck wie die Abgase durchgeführt wird, wodurch ein teilweise flüssiger Carbamatstrom (13) gewonnen wird;
   ii) vollständiges Kondensieren des teilweise flüssigen Carbamatstroms (13), wodurch ein flüssiger Carbamatstrom (15) gewonnen wird;
   iii) Komprimieren des aus Schritt ii) gewonnenen flüssigen Carbamatstroms (15) bei einem Druck größer oder gleich dem Harnstoffsynthesedruck (130 bis 200 bar), wodurch ein flüssiger Strom komprimierten Carbamats (16) gewonnen wird;
   iv) Inkontaktbringen des aus Schritt iii) stammenden flüssigen Stroms komprimierten Carbamats (16) mit dem aus dem Melaminsyntheseverfahren stammenden Abgasstrom (11) mit folgendem Wärmetausch, wobei die durch die Teilkondensation des zugeführten Abgasstroms (11) erzeugte Wärme auf den flüssigen Strom komprimierten Carbamats (16) übertragen wird, und
   v) Zuführen des in Schritt iv) gewonnenen flüssigen Stroms komprimierten und erwärmten Carbamats (20) als Reagenz des Harnstoffsyntheseverfahrens.

2. Verfahren nach Anspruch 1, wobei Schritt ii) zum vollständigen Kondensieren des teilweise flüssigen Carbamatstroms (13) durch Wärmeübertragung auf einen Ammoniakstrom (17), wodurch ein erwärmter Ammoniakstrom (18) und ein flüssiger Carbamatstrom (15) gewonnen werden, oder auf ein Kühlfluid (21), wodurch ein flüssiger Carbamatstrom (15) gewonnen wird, durchgeführt wird.

3. Verfahren nach Anspruch 1, wobei Schritt ii) zum vollständigen Kondensieren des teilweise flüssigen Carbamatstroms (13) durchgeführt wird mittels

   - eines Schritt i'), der eine erste Teilwärmeübertragung auf einen Ammoniakstrom (17) umfasst, wodurch ein erwärmter Ammoniakstrom (18), der dem Harnstoffsyntheseverfahren anschließend als Reagenz (18) zugeführt wird, und ein noch teilweise flüssiger Carbamatstrom (14) gewonnen werden, und
   - einer zweiten Wärmeübertragung von dem noch teilweise flüssigen Carbamatstrom (14) auf ein Kühlfluid (21), wodurch der flüssige Carbamatstrom (15) gewonnen wird.

**4.** Verfahren nach einem oder mehreren der vorhergehenden Ansprüche, wobei die Abgase (11) 0 bis 35 Gew.-% Wasser enthalten und einen Druck im Bereich von 2 bis 120 bar aufweisen.

**5.** Verfahren nach einem oder mehreren der vorhergehenden Ansprüche, ferner umfassend das Zuführen eines aus der Harnstoffanlage stammenden wässrigen Stroms (12) vor dem Schritt ii) zum Kondensieren.

**6.** Verfahren nach Anspruch 5, wobei der wässrige Strom (12) eine aus einem der Zersetzungsabschnitte des Harnstoffverfahrens stammende wässrige Carbamatlösung oder eine aus dem Aufbereitungsabschnitt des Prozesswassers ebenfalls des Harnstoffverfahrens stammende wässrige Carbamatlösung ist.

**7.** Verfahren nach einem oder mehreren der vorhergehenden Ansprüche 2 bis 6, wobei das Zuführen des aus der Harnstoffanlage stammenden wässrigen Stroms (12) vor oder nach dem Schritt i') zum Erwärmen des Ammoniakstroms (17) mit dem Abgasstrom (11, 13) und vor dem Schritt ii) zum Kondensieren erfolgt.

**8.** Vorrichtung zur Integration einer Anlage zur Herstellung von Harnstoff und einer Anlage zur Herstellung von Melamin, die einen Wärmerückgewinnungsblock (1') umfasst, der durch eine Leitung (13') mit einem Kondensationsblock (2') verbunden ist, der wiederum mittels einer Leitung (15') mit einem Kompressionsblock (3') verbunden ist, der wiederum mittels eines Versorgungskanals (16') mit dem Wärmerückgewinnungsblock (1') verbunden ist, wobei der Wärmerückgewinnungsblock (1') durch einen Versorgungskanal (11') von aus der Melaminherstellungsanlage stammenden Abgasen gespeist wird und einen Auslasskanal (20') umfasst, der geeignet ist, um dem Syntheseabschnitt der Harnstoffanlage einen flüssigen Strom komprimierten und erwärmten Carbamats zuzuführen.

**9.** Vorrichtung nach Anspruch 8, ferner umfassend einen Vorwärmer (4'), der in Bezug auf die Strömungsrichtung der Abgase und des Carbamatstroms stromabwärts des Wärmerückgewinnungsblocks (1') angeordnet und mit dem Wärmerückgewinnungsblock (1') mittels eines Kanals (13') zum Zuführen eines teilweise kondensierten Carbamatstroms verbunden ist, wobei der Vorwärmer (4') mittels eines Kanals (17') gespeist wird, der geeignet ist, um einen Ammoniakstrom zuzuführen, und einen Auslasskanal (18') umfasst, der geeignet ist, um dem Harnstoffsyntheseabschnitt den in dem Vorwärmer (4') vorgewärmten Ammoniakstrom zuzuführen, sowie einen Auslasskanal (14'), der geeignet ist, um dem Kondensationsblock (2') einen teilweise flüssigen Carbamatstroms zuzuführen.

**10.** Vorrichtung nach Anspruch 8 oder Anspruch 9, wobei die Vorrichtung einen Versorgungskanal (12') umfasst, der geeignet ist, um einen wässrigen Strom stromaufwärts oder stromabwärts des Wärmerückgewinnungsblocks (1') in Bezug auf die Strömungsrichtung der Abgase und des Carbamatstroms zuzuführen.

**11.** Vorrichtung nach Anspruch 8 und 9, wobei der Versorgungskanal (12'), der geeignet ist, um den wässrigen Strom zuzuführen, stromaufwärts oder stromabwärts des Vorwärmers (4') und stromaufwärts des Kondensators (2') angeschlossen ist.

## Revendications

**1.** Procédé intégré pour la production d'urée et de mélamine comprenant les étapes suivantes :

i) alimenter un flux de gaz résiduels (11) provenant du processus de synthèse de la mélamine dans une étape de condensation partielle et de récupération de chaleur effectuée à la même pression que les gaz résiduels, afin d'obtenir un flux partiellement liquide de carbamate (13) ;
ii) condensation totale du flux partiellement liquide de carbamate (13), pour obtenir un flux liquide de carbamate (15) ;
iii) compression du flux liquide de carbamate (15) obtenu à l'étape ii) à une pression égale ou supérieure à la pression de synthèse de l'urée (130-200 bars) pour obtenir un flux liquide de carbamate comprimé (16) ;
iv) le flux liquide de carbamate comprimé (16) provenant de l'étape iii) est mis en contact avec le flux d'effluents gazeux (11) provenant du processus de synthèse de la mélamine avec un échange de chaleur conséquent dans lequel la chaleur produite par la condensation partielle du flux d'effluents gazeux (11) alimenté est transférée au flux liquide de carbamate comprimé (16) et ;
v) alimenter ledit flux liquide de carbamate (20) comprimé et chauffé obtenu à l'étape iv) en tant que réactif du processus de synthèse de l'urée.

**2.** Procédé selon la revendication 1, dans lequel l'étape ii) pour la condensation totale du flux partiellement liquide de

carbamate (13) est effectuée par transfert de chaleur à un flux d'ammoniac (17), obtenant un flux chauffé d'ammoniac (18) et un flux liquide de carbamate (15) ou à un fluide de refroidissement (21), obtenant un flux liquide de carbamate (15).

**3.** Procédé selon la revendication 1, dans lequel l'étape ii) pour la condensation totale du flux partiellement liquide de carbamate (13) est réalisée au moyen de

- une étape i') qui comprend un premier transfert partiel de chaleur à un courant d'ammoniac (17), obtenant un courant d'ammoniac chauffé (18), alimenté ensuite comme réactif (18) dans le procédé de synthèse de l'urée, et un courant encore partiellement liquide de carbamate (14) ; et
- un second transfert de chaleur du flux de carbamate encore partiellement liquide (14) vers un fluide de refroidissement (21), obtenant le flux de carbamate liquide (15).

**4.** Procédé selon une ou plusieurs des revendications précédentes, dans lequel les effluents gazeux (11) contiennent de 0 à 35 % en poids d'eau et ont une pression comprise entre 2 et 120 bars.

**5.** Procédé selon l'une ou plusieurs des revendications précédentes, comprenant également l'alimentation d'un flux aqueux (12) provenant de l'usine d'urée, en amont de l'étape de condensation ii).

**6.** Procédé selon la revendication 5, dans lequel le flux aqueux (12) est une solution aqueuse de carbamate provenant de l'une des sections de décomposition du procédé d'urée, ou une solution aqueuse de carbamate provenant de la section de traitement de l'eau de traitement, là encore du procédé d'urée.

**7.** Procédé selon une ou plusieurs des revendications précédentes de 2 à 6, dans lequel l'alimentation du flux aqueux (12) provenant de l'usine d'urée a lieu en amont ou en aval de l'étape i') de chauffage du flux d'ammoniac (17) avec le flux d'effluents gazeux (11, 13), et en amont de l'étape de condensation ii).

**8.** Appareil pour l'intégration d'une installation de production d'urée et d'une installation de production de mélamine qui comprend un bloc de récupération de chaleur (1'), connecté par une conduite (13') à un bloc de condensation (2'), à son tour connecté par une conduite (15') à un bloc de compression (3'), à son tour connecté par une conduite d'alimentation (16') au bloc de récupération de chaleur (1') ledit bloc de récupération de chaleur (1') étant alimenté par un conduit d'alimentation (11') en effluents gazeux provenant de l'usine de production de mélamine et comprenant un conduit de sortie (20') adapté à l'alimentation d'un flux liquide de carbamate comprimé et chauffé vers la section de synthèse de l'usine d'urée.

**9.** Appareil selon la revendication 8, comprenant également un préchauffeur (4'), disposé en aval du bloc de récupération de chaleur (1'), par rapport au sens d'écoulement des effluents gazeux et du flux de carbamate, et connecté audit bloc de récupération de chaleur (1') au moyen d'un conduit (13') pour l'alimentation d'un flux de carbamate partiellement condensé, ledit préchauffeur (4') étant alimenté au moyen d'un conduit (17') adapté à l'alimentation d'un flux d'ammoniac et comprenant un conduit de sortie (18') adapté à l'alimentation du flux d'ammoniac préchauffé dans ledit préchauffeur (4') vers la section de synthèse de l'urée et un conduit de sortie (14') adapté à l'alimentation d'un flux partiellement liquide de carbamate vers le bloc de condensation (2').

**10.** Appareil selon la revendication 8 ou la revendication 9, dans lequel l'appareil comprend un conduit d'alimentation (12') adapté à l'alimentation d'un flux aqueux en amont ou en aval du bloc de récupération de chaleur (1'), par rapport à la direction d'écoulement des effluents gazeux et du flux de carbamate.

**11.** Appareil selon les revendications 8 et 9, dans lequel le conduit d'alimentation (12') apte à alimenter le flux aqueux est connecté en amont ou en aval du préchauffeur (4') et en amont du condenseur (2').

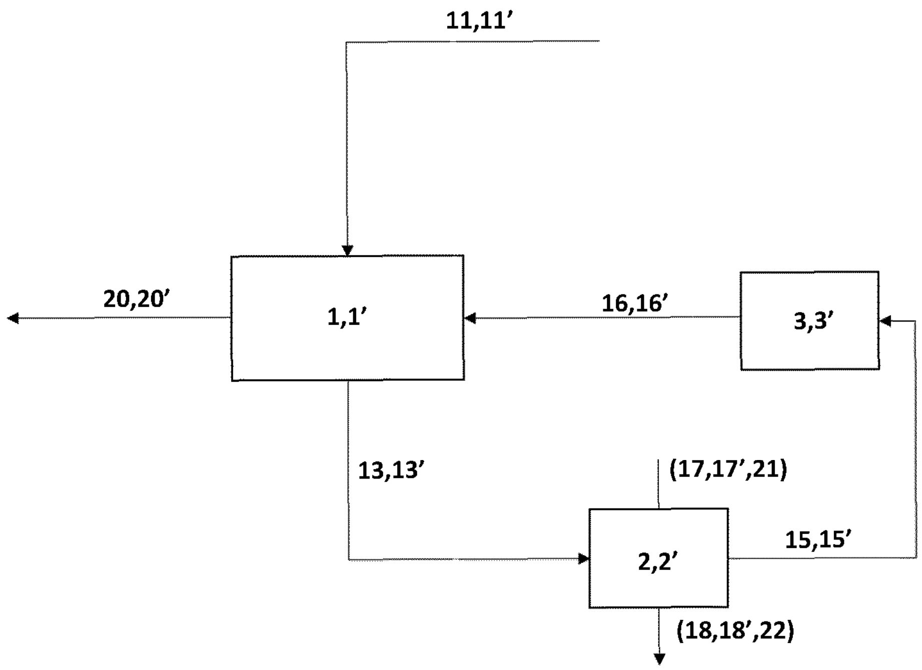
11,11'
20,20'
1,1'
16,16'
3,3'
13,13'
(17,17',21)
2,2'
15,15'
(18,18',22)

Fig. 1

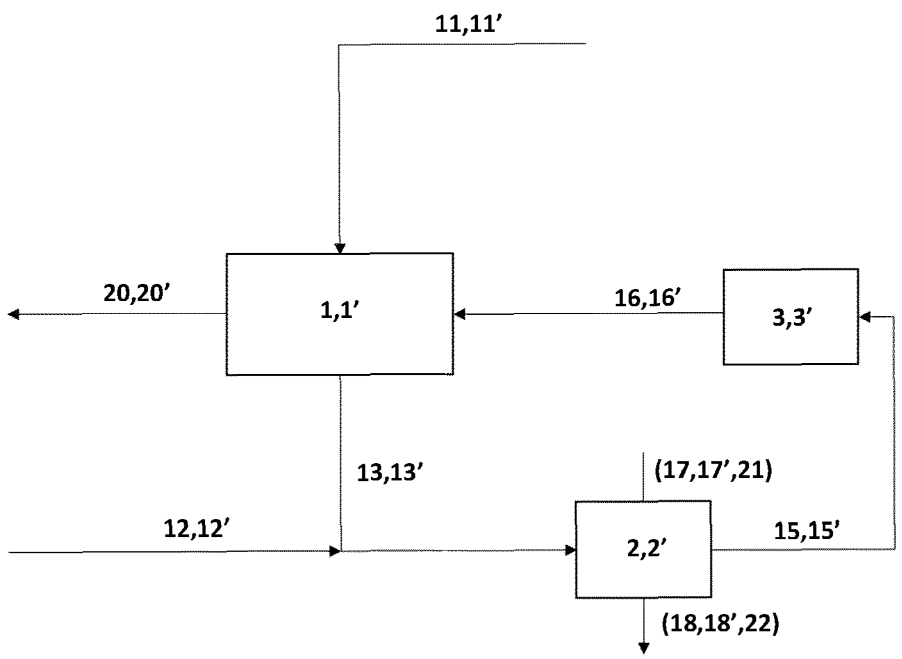
11,11'
20,20'
1,1'
16,16'
3,3'
13,13'
(17,17',21)
12,12'
2,2'
15,15'
(18,18',22)

Fig. 2

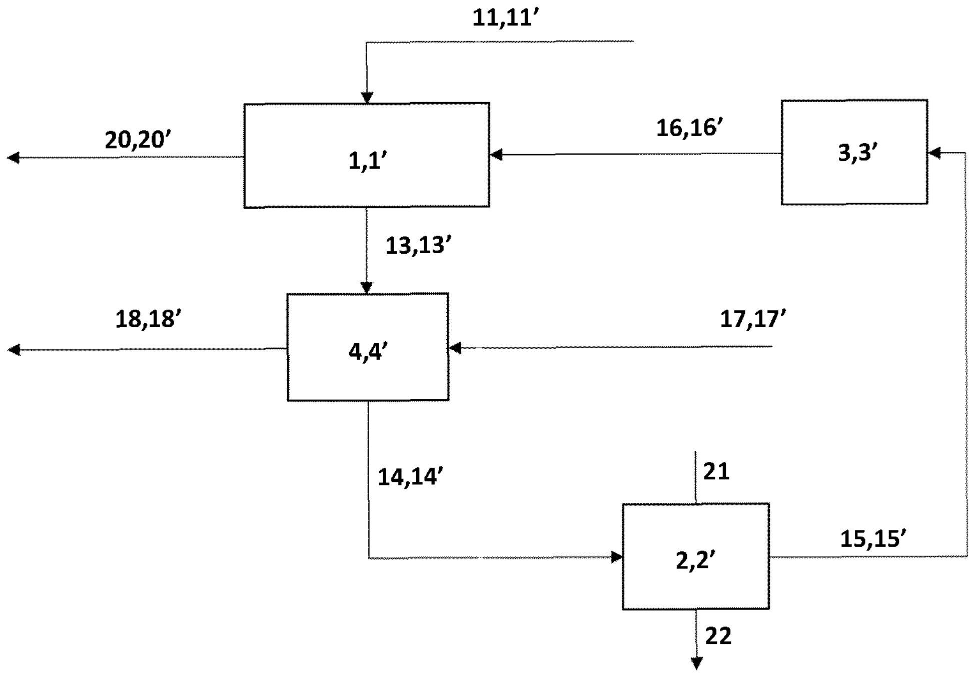
11,11'
20,20'
1,1'
16,16'
3,3'
13,13'
18,18'
4,4'
17,17'
14,14'
21
2,2'
15,15'
22

Fig. 3

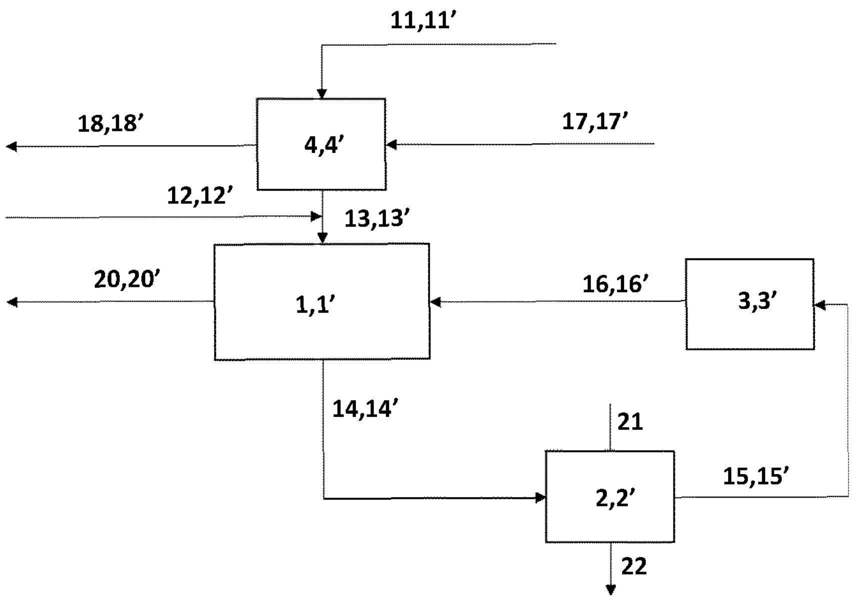
11,11'
18,18'
4,4'
17,17'
12,12'
13,13'
20,20'
1,1'
16,16'
3,3'
14,14'
21
2,2'
15,15'
22

Fig. 4

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- EP 1716111 A
- EP 3083571 A
- WO 2016171562 A1